# EUROPEAN PATENT APPLICATION

(11) **EP 4 410 947 A2**
(43) Date of publication of application: **07.08.2024**
(21) Application number: 24176365.5
(22) Date of filing: 28.05.2020
(51) Int. Cl.: C12M 1/00

(54) **CELL PRODUCTION DEVICE AND SYSTEM THEREFOR**

(30) Priority: 29.08.2019 US 201962893594 P
(62) Divisional of application: 20857800.5
(71) Applicant: FANUC CORPORATION, Oshino-mura Minamitsuru-gun Yamanashi 401-0597 (JP); I Peace, Inc., Palo Alto, CA 94303 (US)
(72) Inventor: BAN, Kazunori, Yamanashi, 401-0597 (JP); KINOSHITA, Satoshi, Yamanashi, 401-0597 (JP); TANABE, Koji, Palo Alto, 94303 (US); HIRAIDE, Ryoji, Kyoto-shi, 6068414 (JP)
(74) Representative: Schmidt, Steffen J.

(57) **Abstract**

This cell production device comprises: a cell production plate that includes a fluid circuit having a plurality of functional parts integrated therein; and a plurality of closed type connectors that connect the fluid circuit to an external space in a closed manner. The fluid circuit comprises, as the plurality of functional parts: a plurality of injection and discharge units capable of injecting or discharging a plurality of types of fluids into or out of the fluid circuit via the plurality of closed type connectors; a plurality of fluid reservoir units capable of storing the plurality of types of fluids to be injected or discharged; and a cell induction and culture unit that performs at least one of induction and culture of cells based on the stored plurality of types of fluids.

## Description

### FIELD

The present invention relates to a cell production device and a system therefor, and particularly to a cell production device that simplifies a fluid injection and discharge process and a system therefor.

### BACKGROUND

Embryonic stem cells (ES cells) are stem cells established from early embryos of human or mouse, and they have pluripotency to differentiate into all cells present in the body. Human ES cells are considered to be applicable for cell transplantation for many diseases such as Parkinson's disease, juvenile diabetes, and leukemia. However, ES cell transplantation, like organ transplantation, has a problem of inducing rejection. From an ethical standpoint, there are many objections to the utilization of ES cells, which are established by destroying human embryos.

To address this issue, Professor Shinya Yamanaka of Kyoto University succeeded in establishing induced pluripotent stem cells (iPS cells) by introducing four genes: Oct3/4, Klf4, c-Myc, and Sox2 into somatic cells, and was awarded the 2012 Nobel Prize in Physiology or Medicine (see, for example, Patent literature 1). iPS cells are ideal pluripotent cells with no rejection or ethical issues, and are expected to be utilized in cell transplantation methods.

Induced stem cells, such as iPS cells, are established by introducing an inducing factor, such as a gene, into the cells, which are expansively cultured and cryopreserved. However, for preparing iPS cells for clinical use (GLP, GMP grade) or the like, for example, a clean room that is kept very clean is required, which involves high maintenance costs. For industrialization, how to improve the efficiency of clean room operation methods to reduce costs has been a problem.

Although preparation of iPS cells is mostly performed manually, few technicians are capable of producing iPS cells for clinical use. The series of operations from establishment to storage of stem cells are complicated, which is a problem. Cell culture for clinical use requires three steps: confirmation of standard of process (SOP), manipulation according to SOP, and confirmation whether or not the manipulation was performed according to SOP, and it is very unproductive for a person to perform these steps. Cell culture needs to be managed 24 hours a day, every day, and stem cells can be stored for decades, which is beyond the capacity of human resources alone to manage.

Therefore, closed system cell production devices that do not require a highly clean room and can be operated in a normally controlled area (for example, grade D level or higher for at least one of microorganisms and particulates in WHO-GMP standards) have been developed (see, for example, Patent literature 2). In order to automate complex cell production processes by also eliminating human resources, cell production systems equipped with robots to assist in cell production have also been developed. The following documents are known as prior art concerning such cell production devices.

Patent literature 3 discloses a somatic cell production system that packages a pre-introduction cell delivery channel, a factor introduction device that prepares inducing factor-introduced cells by introducing somatic cell inducing factors into pre-introduction cells, and a cell production device that prepares somatic cells by culturing inducing factor-introduced cells in a single housing.

Patent literature 4 discloses a cell culture container with a closed system of culture containers and flow paths, in which the growth state of the cell culture can be clearly observed because the cell culture container holds the second container eccentrically inside the first container.

Patent literature 5 discloses a cell culture device in which a culture medium storing means, a cell inoculation means, and a culture container are configured in a closed system. The cell culture device determines the culture status of cells based on images of cells in the culture container, and performs culture operations based on this determination, which saves the operator's labor.

Patent literature 6 discloses a cell culture device comprising a main body with side walls enclosing the volume of a cell culture chamber, a lid covering the cell culture chamber, and a bottom plate arranged at the bottom of the main body, wherein microfluidic conduits are integrally formed on the main body to provide fluid communication between inlet and outlet connectors and the cell culture chamber.

Patent literature 7 discloses a microchip reaction device comprising a bubble removal means that moves bubbles in the internal space of the microchip to the external space. Patent literature 8 discloses a cell culture device with air vents that allow gas to be discharged from a first culture solution storage chamber and a second culture solution storage chamber, wherein the vents are equipped with air filters.

Patent literature 9 discloses a cell culture device comprising a flow path integrated plate and a base plate. The flow path integrated plate includes a flow path plate in which a flow path for a culture solution is formed, and a pump unit in which a group of peristaltic pumps are arranged to supply and discharge the culture solution, and the base plate includes a drive source such as a motor.

### CITATION LIST

Patent literature 1: Japanese Patent 4183742
Patent literature 2: Japanese Unexamined Patent Publication No. 2018-019685
Patent literature 3: WO 2018/154788
Patent literature 4: WO 2014/049701
Patent literature 5: WO 2007/052716
Patent literature 6: Japanese Unexamined Patent Application Publication (Translation of PCT Application) No. 2014-514926
Patent literature 7: Japanese Unexamined Patent Publication No. 2014-226623
Patent literature 8: WO 2017/154880
Patent literature 9: Japanese Unexamined Patent Publication No. 2017-221166

### SUMMARY

### TECHNICAL PROBLEM

Most of conventional cell culture devices integrate only cell culture functions, such as culture medium storing reservoirs, culture medium supply and discharge flow paths, and cell culture containers, but few integrate also cell induction functions, such as cell initiation, reprogramming, fate diversion, direct reprogramming, differentiation diversion, differentiation induction, and transformation by introducing an inducing factor. In a device in which these various functional sites are configured in a closed system, various fluids need to be injected or discharged into the device in a predetermined amount at an appropriate timing, which makes the injection and discharge process laborious. If cell production using such a device is to be fully automated, the injection and discharge operations of the robot will also be complicated.

Therefore, there is a need for a technique to simplify fluid injection and discharge processes in a cell production device that integrates a plurality of functional sites.

### SOLUTION TO PROBLEM

One aspect of the present disclosure provides a cell production device comprising: a cell production plate that includes a fluid circuit in which a plurality of functional sites are integrated; and a plurality of closed type connectors that connects the fluid circuit to an external space in a closed manner, wherein the fluid circuit comprises a plurality of injection and discharge units capable of injecting or discharging a plurality of types of fluids into or out of the fluid circuit via a plurality of closed type connectors, a plurality of fluid reservoir units capable of storing a plurality of types of fluids to be injected or discharged, and a cell induction and culture unit that performs at least one of induction and culture of cells based on the stored plurality of types of fluids.

Another aspect of the present disclosure provides a cell production device comprising a cell production plate that includes a fluid circuit in which a plurality of functional sites are integrated, wherein the fluid circuit comprises, as the plurality of functional sites, a plurality of fluid reservoir units capable of storing a plurality of types of fluids and a cell induction and culture unit that performs at least one of induction and culture of cells based on the plurality of types of stored fluids, wherein the fluid reservoir unit stores at least one of a fluid containing source cells, a reagent for cell separation, a reagent for introducing an inducing factor, a culture medium for initialization or induction, and a fluid containing target cells.

Another aspect of the present disclosure provides a cell production system comprising: a cell production device comprising a cell production plate that includes a fluid circuit integrating a plurality of functional sites, and a plurality of closed type connectors that connects the fluid circuit to an external space in a closed manner; and a fluid container capable of being connected to at least one of the plurality of closed type connectors, wherein the fluid circuit comprises a plurality of injection and discharge units capable of injecting or discharging a plurality of types of fluids into or out of the fluid circuit via a plurality of closed type connectors, and wherein the fluid container comprises a fluid reservoir unit capable of storing at least one of the plurality of types of fluids to be dispensed or suctioned, and a dispense and suction unit capable of dispensing or suctioning fluids into or out of the fluid circuit via at least one of the plurality of closed type connectors.

### ADVANTAGEOUS EFFECTS OF INVENTION

According to the present disclosure, since a cell production plate is capable of storing a plurality of types of fluids, or a fluid container is capable of dispensing a stored fluid to a cell production plate, it is possible to utilize a predetermined amount of a specific fluid at an appropriate timing even in a cell production device integrating a plurality of functional sites, thereby simplifying a fluid injection and discharge process by human resources or robots.

### BRIEF DESCRIPTION OF DRAWINGS

FIG. 1 is a configuration diagram of a cell production device in one embodiment.
FIG. 2 is an exploded perspective view of one example of a cell production plate.
FIG. 3A is an enlarged sectional view of one example of a method of fixing a flat plate and a lid.
FIG. 3B is an enlarged sectional view of one example of a method of fixing a flat plate and a lid.
FIG. 4A is a configuration diagram illustrating one example of a fluid reservoir unit provided in a cell production plate.
FIG. 4B is a configuration diagram showing one example of a fluid reservoir unit in a fluid container.
FIG. 4C is a configuration diagram showing one example of a fluid reservoir unit in a fluid container.
FIG. 5A is a frontal perspective view illustrating one example of a base plate.
FIG. 5B is a back perspective view illustrating one example of a base plate.

### DESCRIPTION OF EMBODIMENTS

Embodiments of the present disclosure will be described in detail with reference to the accompanying drawings. In each drawing, the same or similar symbols are assigned to the same or similar components. The embodiments described below are not intended to limit the technical scope of the invention and the meaning of the terms in claims. The term "closed" in this document means that sources of contamination, such as microorganisms or viruses, do not enter the device and cause biological contamination, and/or that fluids (including substances such as cells, microorganisms, viral particles, proteins, and nucleic acids) inside the device does not leak and cause cross-contamination, and/or that handling donor fluids infected with pathogens inside the device does not create a biohazard. Note, however, that the device herein may be configured to allow fluids that are not sources of contamination, such as carbon dioxide, nitrogen, or oxygen, to enter or leak outside the device.

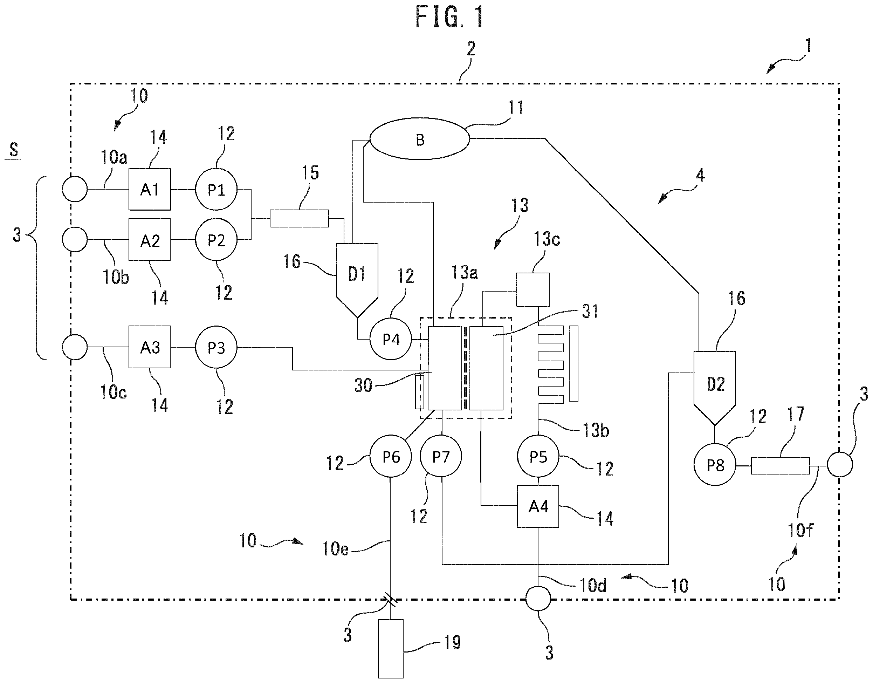

FIG. 1 illustrates the configuration of a cell production device 1 in the present embodiment. The cell production device 1 is a cell conversion device with a cell induction function that performs cell initialization, reprogramming, fate diversion, direct reprogramming, differentiation diversion, differentiation induction, transformation, or the like by introducing an inducing factor, or may be a cell culture device that only has a cell culture function merely performing culture, expansion culture, or the like. The cell production device 1 injects a fluid containing source cells (for example, somatic cells such as blood cells or fibroblasts, or stem cells such as ES cells or iPS cells), produces target cells (for example, stem cells, progenitor cells, or final differentiated cells) from the source cells, and discharges the fluid containing the target cells. As the target cells, differentiated cells such as fibroblasts, neural cells, retinal epithelial cells, hepatocytes, β cells, renal cells, mesenchymal stem cells, blood cells, megakaryocytes, T cells, chondrocytes, cardiomyocytes, myocytes, vascular cells, epithelial cells, or other somatic cells may be prepared. The cell production device 1 is a closed system cell processing device that integrates all parts that should be highly clean inside, and can be used in normally controlled areas. The closed space inside the device is configured in such a manner that gases, viruses, microorganisms, impurities, or the like are not exchanged with the outside. Note, however, that the device may be configured to allow exchange of non-contaminant fluids between the inside and outside of the device by additionally providing the device with a fluid exchange filter, or the like, as described below.

As illustrated in FIG. 1, the cell production device 1 includes a cell production plate 2 and a closed type connector 3. The cell production plate 2 includes a fluid circuit 4 in a closed system that is shut off from an external space S, and the fluid circuit 4 includes a flow path that highly integrates a plurality of functional sites. The closed type connector 3 is a connector for injecting a fluid into the fluid circuit 4 or for discharging a fluid from the fluid circuit 4, and is attached to the cell production plate 2. The closed type connector 3 is a connector that connects the fluid circuit 4 and a fluid container to the external space S in a closed manner, and may be, for example, a sterile connection connector, a needleless connector, a needle connector, or a heat-fused tube. The needleless connector may be a split septum type or a mechanical valve type. The fluid container is a syringe, variable volume bag, or the like, and when the closed type connector 3 is connected to the fluid container, the fluid circuit 4 is in fluid communication with the fluid container, while when the closed type connector 3 is not connected to the fluid container, the fluid circuit 4 is shut off from the external space S. This prevents biological contamination, cross-contamination, and biohazard of the fluid circuit 4. In order to enable injection or discharge of a plurality of types of fluids, the cell production device 1 preferably includes a plurality of closed type connectors 3.

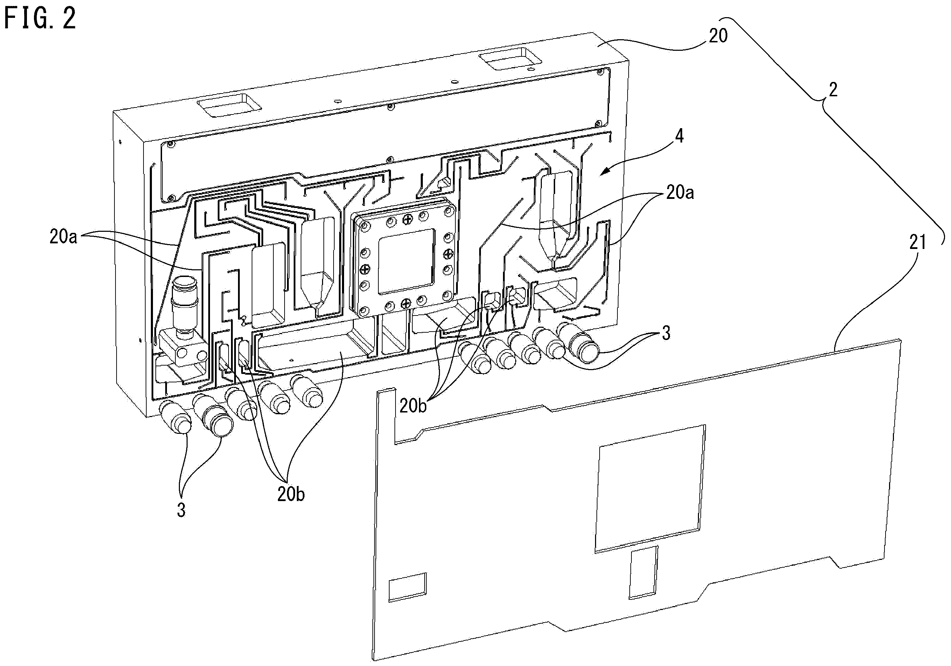

FIG. 2 illustrates one example of a cell production plate 2. As illustrated in FIG. 2, the cell production plate 2 includes a flat plate 20 and a lid 21. The flat plate 20 can be molded from, for example, a biologically safe resin or metal. The flat plate 20 is preferably molded by a molding process using a mold, such as injection molding or compression molding, and the flat plate 20 may also be molded using a 3D printer or the like. A 3D printer can employ a variety of molding methods, such as optical molding, thermal dissolution layering, powder sintering, or inkjet. On at least one of the surface and the back of the flat plate 20, a groove 20a is provided as a flow path for fluid flow, and a plurality of grooves 20a are combined to form the fluid circuit 4. A portion of the fluid circuit 4 is provided with a groove 20a that is relatively larger in width or depth to form a reservoir tank 20b for temporary storage of fluid. Walls of the groove 20a and reservoir tank 20b may be coated with poly-HEMA (poly 2-hydroxyethyl methacrylate) to make the walls non-adhesive to cells. Conversely, when cells have difficulty entering the groove 20a or reservoir tank 20b, walls of the groove 20a and reservoir tank 20b may be made low protein adsorbent. At least a portion of the grooves 20a and reservoir tanks 20b are preferably white or black in order to observe changes over time in fluid, cells, cell masses, or the like by image recognition sensors, ultrasonic recognition sensors, or the like.

The lid 21 may be made of, for example, a biologically safe resin, quartz glass, or the like. The lid 21 (or at least a portion of the cell production plate 2) is preferably transparent in order to observe changes over time of fluid, cells, cell masses, or the like in the fluid circuit 4 by image recognition sensors, ultrasonic recognition sensors, or the like. This observation enables transition to the next cell production process when appropriate. The lid 21 is fixed to the flat plate 20 in such a manner that the lid covers the fluid circuit 4 by a biologically safe fixing method, such as chemical bonding, weld bonding, or adhesive bonding, in order to shut off the fluid circuit 4 from the external space. For chemical bonding, silane coupling agents, plasma irradiation, or the like may be used. For weld bonding, laser welding, ultrasonic welding, or the like may be used, and for adhesive bonding, UV-curing adhesives or the like may be used. After fixing the flat plate 20 and the lid 21, the cell production plate 2 is subjected to sterilization, such as heat sterilization, gamma ray sterilization, UV sterilization, or electron beam sterilization, to make the fluid circuit 4 highly clean.

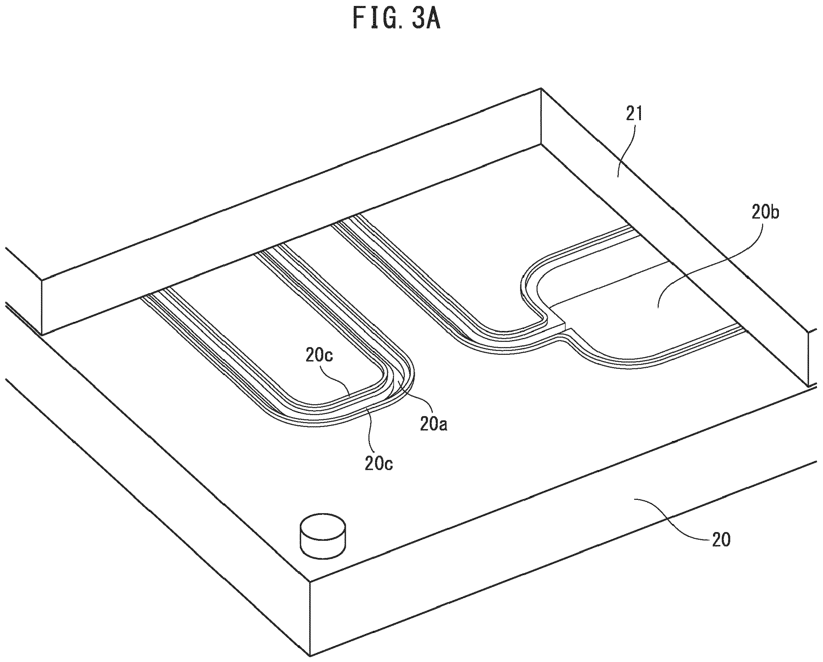

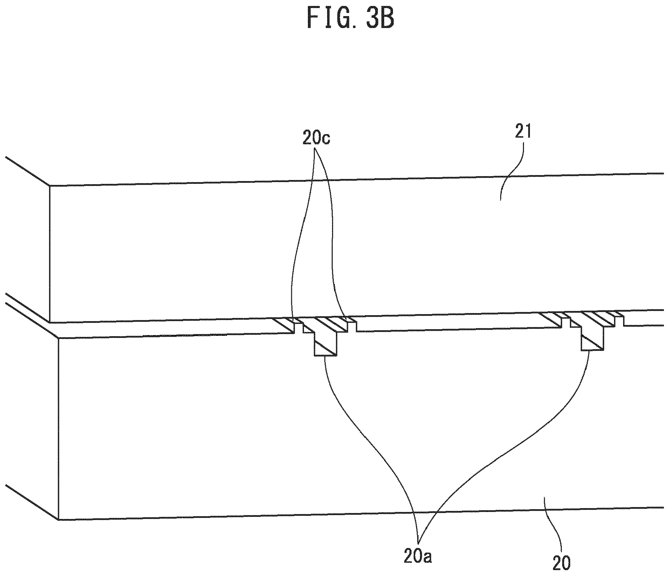

FIG. 3A and FIG. 3B illustrate one example of a method of fixing a flat plate and a lid. In order to shut off the fluid circuit 4 from the external space, banks 20c may be formed in advance on both sides of the groove 20a and the reservoir tank 20b, the flat plate 20 may be covered with the lid 21, and the groove 20a and the reservoir tank 20b may be sealed by heating the banks 20c by irradiating or applying laser light, ultrasonic waves, or the like to at least the banks 20c and welding the flat plate 20 and the lid 21. Alternatively, the lid 21 may be applied to the flat plate 20 including the groove 20a and the reservoir tank 20b, and at least the portions other than the groove 20a and the reservoir tank 20b may be heated by irradiating or applying laser light, ultrasonic waves, or the like, and the groove 20a and the reservoir tank 20b may be sealed by welding the flat plate 20 and the lid 21. In this case, all portions other than the groove 20a and the reservoir tank 20b are fixed, thus increasing the strength of the fixation.

Referring again to FIG. 1, the fluid circuit 4 includes at least an injection and discharge unit 10 and a cell induction and culture unit 13 as a plurality of functional sites. The fluid circuit 4 may optionally include a first variable volume unit 11, a transfer unit 12, a fluid reservoir unit 14, a fluid mixing unit 15, a cell separation unit 16, and a cell mass disruption unit 17. Since these variety of functional sites are integrated in one cell production plate 2, manufacturing processes such as closed connection of separate parts via tubes, pumps, connectors, and the like are unnecessary, thereby reducing the man-hours and manufacturing cost of the cell production device 1.

The injection and discharge unit 10 includes an injection and discharge channel that injects or discharges fluid into or out of the fluid circuit 4 via the closed type connector 3. In order to enable a plurality of types of fluids to be injected or discharged, the injection and discharge unit 10 includes a plurality of injection and discharge channels 10a-10f For example, the first injection and discharge channel 10a is capable of injecting or discharging fluids containing source cells and the like, while the second injection and discharge channel 10b is capable of injecting or discharging fluids such as reagents for source cell separation, anticoagulants, or phosphate-buffered saline. A third injection and discharge channel 10c is capable of injecting or discharging fluids such as inducing factor introduction reagents, and a fourth injection and discharge channel 10d is capable of injecting or discharging a variety of culture media such as initialization or induction medium, cell detachment reagents such as trypsin alternative recombinant enzymes, or fluids such as single cell separation reagents. Examples of the culture medium for induction includes initialization medium, reprogramming medium, fate diversion medium, direct programming medium, differentiation diversion medium, differentiation induction medium, and transformation medium. Furthermore, a fifth injection and discharge channel 10e is capable of discharging or injecting fluid containing cells that have undergone at least one of induction and culture into the fluid container 19 as a sample, and a sixth injection and discharge channel 10f is capable of discharging or injecting fluid containing target cells into the fluid container. The fluid container 19 for sample discharge may be a closed type connector 3, such as a variable volume bag that connects to a heat-fused tube. When discharging a fluid containing target cells, a refrigerant such as liquid nitrogen may be supplied around the sixth injection and discharge channel 10f to freeze the fluid containing the target cells and seal the cell production plate, or the fluid container into which the fluid is discharged from the sixth injection and discharge channel 10f via the closed type connector 3 may be frozen with a refrigerant such as liquid nitrogen.

The first variable volume unit 11 includes a physical or chemical variable volume material that stores a fluid that is extruded or withdrawn by injected or discharged fluid. A fluid relief flow path is provided to allow a fluid originally contained in the fluid circuit 4 to escape, and either a physical variable volume material is connected to the fluid relief flow path or a chemical variable volume material is placed in a reservoir tank with a pressure valve that opens and closes at a certain pressure in the fluid relief flow path, to allow fluid movement while keeping the fluid circuit 4 sealed. The physical variable volume material may be, for example, a flexible bag, or a syringe. The chemical variable volume material may include, for example, a fluid absorbent such as soda lime, or silica gel, and a fluid releaser that is placed in a different reservoir tank than the reservoir tank in which the fluid absorbent is placed. The variable volume material keeps the internal pressure of the closed fluid circuit 4 approximately constant, and the fluid extruded or withdrawn by the injected or discharged fluid is confined in the cell production plate 2. Therefore, there is no need to release fluid outside the cell production plate 2 or to take in fluid from outside, and the cell production plate 2 can be formed into a plate while maintaining airtightness of the fluid circuit 4. Such a cell production plate 2 is easy for a robot to handle.

The transfer unit 12 includes a pump that transfers a fluid in the fluid circuit 4. The pump can be a flow-controllable positive displacement pump, such as a rotary pump or a reciprocating pump. As the rotary pump, a peristaltic pump is preferred. In the case of a peristaltic pump, flexible tubing is hermetically connected to a connector at the end of the flow path, and fluid is transferred by squeezing the tubing with rollers. Since the tubing is blocked by the rollers, when the pump is stopped, the fluid flow is blocked and the flow rate can be controlled. As the reciprocating pump, a diaphragm pump is desirable. Note, however, that in the case of a diaphragm pump, since the diaphragm does not shut off the flow path, flow control is possible by using a flow shutoff valve in combination.

In order to transfer a fluid to an appropriate functional site at an appropriate time, the transfer unit 12 preferably includes a plurality of pumps P1-P8. For example, the first pump P1 - the third pump P3 transfer a fluid stored in the fluid reservoir units A1 - A3 at an appropriate timing, and the fourth pump P4 and the eighth pump P8 transfer the fluid stored in the cell separation unit 16 at an appropriate timing. The fifth pump P5 transfers a fluid stored in the fluid reservoir unit A4 at an appropriate timing, and the sixth pump P6 - the seventh pump P7 transfer a fluid stored in the cell induction and culture unit 13 at an appropriate timing. When, for example, a peristaltic pump is used as a pump, a rotary encoder capable of detecting the amount of rotation may be provided on the rotation main shaft of the pump in order to obtain information on whether the pump is operating properly, such as whether the pump has reliably rotated or has rotated by an appropriate angle. Alternatively, for example, a visual mark may be provided at the end of the rotation main shaft of the pump, and the rotational movement of the mark may be captured directly in an image by an image recognition sensor. A flow rate measurement unit (not illustrated) may be further provided in the stage before or after the pump to confirm that the pump is pumping reliably. The flow rate measurement unit may be, for example, a flow rate sensor provided adjacent to at least one of a flow path and a reservoir tank connected to a transfer unit, or an image recognition sensor that captures images of fluid changes over time in at least one of a flow path and a reservoir tank connected to the transfer unit. The flow rate sensor can employ a variety of measurement methods that do not adversely affect cells, such as the Kalman vortex type, impeller type, or diaphragm type, and directly obtains flow rate information of the fluid. The image recognition sensor obtains flow rate information from the movement of the fluid by image recognition from an external camera or the like via the transparent lid 21. The image recognition sensor may be diverted from other image recognition sensors herein, which reduces the number of parts and production cost.

The cell induction and culture unit 13 includes a cell induction and culture tank 13a, which performs at least one of cell induction and culture based on a transferred fluid, and a culture medium circulation path 13b, which is in fluid communication with the cell induction and culture tank 13a and circulates the culture medium. The cell induction and culture tank 13a is warmed to a predetermined culture temperature, for example 37°C, by a warming element. The culture medium circulation path 13b is cooled by a cooling element to a predetermined culture medium quality maintenance temperature, for example, 4°C-8°C. The cell induction and culture tank 13a may be sealed and may not be supplied with fluids such as carbon dioxide, nitrogen, and oxygen, but at least one of the cell induction and culture tank 13a and the culture medium circulation path 13b may further include a fluid exchange filter that exchanges fluids such as carbon dioxide, nitrogen, and oxygen inside and outside the device. The cell induction and culture tank 13a may be a three-dimensional culture tank for cell suspension culture, or may be a two-dimensional culture tank for adhesion culture. For adhesion culture, the cell induction and culture tank 13a may be coated with a cell adhesion coating such as matrigel, collagen, polylysine, fibronectin, vitronectin, gelatin, and laminin, laminin fragments, or may be filled with hollow fibers. Furthermore, the cell induction and culture tank 13a may integrally include a culture tank 30 and a culture medium tank 31 that supplies culture medium to the culture tank 30. In this case, the cell induction and culture tank 13a preferably includes a specific component-permeable member 32, for example, a semipermeable membrane, which allows only specific components to pass between the culture tank 30 and the culture medium tank 31. The specific component-permeable member 32 permeates, for example, specific components such as a variety of culture media, coating agents for cell adhesion, and reagents for cell separation.

The cell induction and culture unit 13 may further include a pH measurement unit 13c for measuring the pH value of culture medium used. The pH measurement unit 13c is preferably provided in the culture medium circulation path 13b or the cell induction and culture tank 13a for measuring the pH value of culture medium used. The pH measurement unit 13c may be, for example, an image recognition sensor or an electrode measurement sensor. The image recognition sensor is used for hue measurement for pH values with an external camera or the like via a transparent lid. The electrode measurement sensor measures the pH value by the glass electrode method. In the case of hue measurement, at least a portion of the culture medium circulation path 13b (for example, the bottom of the hue measurement point) can be made white to allow accurate detection of the hue. This pH value makes it possible to quantitatively grasp the condition of the culture medium. In order to sharpen the contour of cell images on the image, the cell induction and culture tank 13a preferably further includes an illumination unit that illuminates the cell induction and culture tank 13a from at least one of the following directions: in front, in the surrounding direction (for example, perpendicular to the observation plane), and in the rear. The illumination unit includes, for example, LED illumination, and may be embedded inside the cell production plate 2, or may be provided outside the cell production plate 2 by making the cell induction and culture tank 13a more convex than the cell production plate 2. To allow illumination to reach cells, the culture tank 30 may be covered with a transparent material that allows light to pass through.

The fluid reservoir unit 14 includes a reservoir tank for storing a fluid to be injected into or discharged out of the fluid circuit 4. In order to enable storage of a plurality of types of fluids, the fluid reservoir unit 14 preferably includes a plurality of reservoir tanks A1-A4. The reservoir tanks A1-A4 are formed as locations where the width or depth of the flow path is relatively increased, enabling a variety of fluids to be utilized in predetermined amounts at appropriate timing. For example, the first reservoir tank A1 stores fluid containing source cells and the like, the second reservoir tank A2 stores fluids such as reagents for source cell separation, anticoagulants, and phosphate-buffered saline, the third reservoir tank A3 stores fluids such as reagents for inducing factors, and the fourth reservoir tank A4 stores fluids such as a variety of culture media, cell adhesion coating agents, and reagents for cell detachment. A reservoir tank for storing fluids containing target cells, or the like, may also be provided.

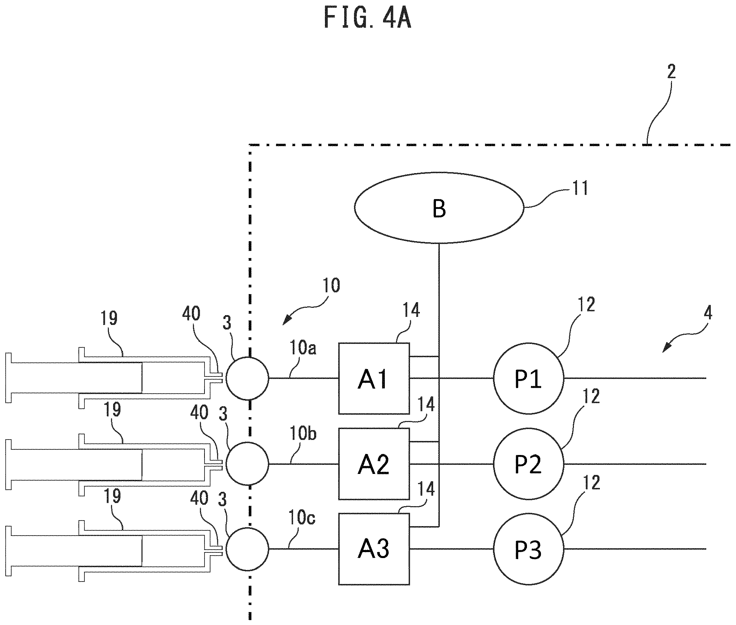

FIG. 4A illustrates one example of the fluid reservoir unit 14 provided in the cell production plate 2. As described above, a plurality of injection and discharge units 10 and a plurality of fluid reservoir units 14 are provided in the cell production plate 2 to enable a plurality of fluids to be injected from the fluid container 19 into the fluid circuit 4 or to be discharged from the fluid circuit 4 into the fluid container 19, respectively, via a plurality of closed type connectors 3, and to be stored in the fluid reservoir unit 14. By providing a plurality of transfer units 12 in the fluid circuit 4 and enabling transfer of a plurality of stored fluids in the fluid circuit 4, it becomes possible to transfer a specific stored fluid in a predetermined amount at an appropriate timing. This can simplify the process of injecting and discharging fluids by human resources or robots. Furthermore, by making these fluid reservoir units 14 in fluid communication with the first variable volume unit 11 and extruding the fluid originally contained in the fluid reservoir units 14 into the first variable volume unit 11 or withdrawing the fluid originally contained in the first variable volume unit 11 into the fluid reservoir units 14, fluid can be transferred within the fluid circuit 4 while maintaining the air tightness of the fluid circuit 4.

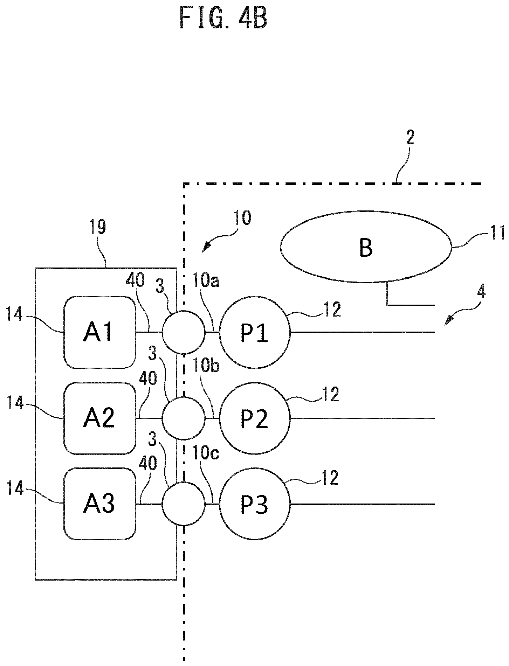

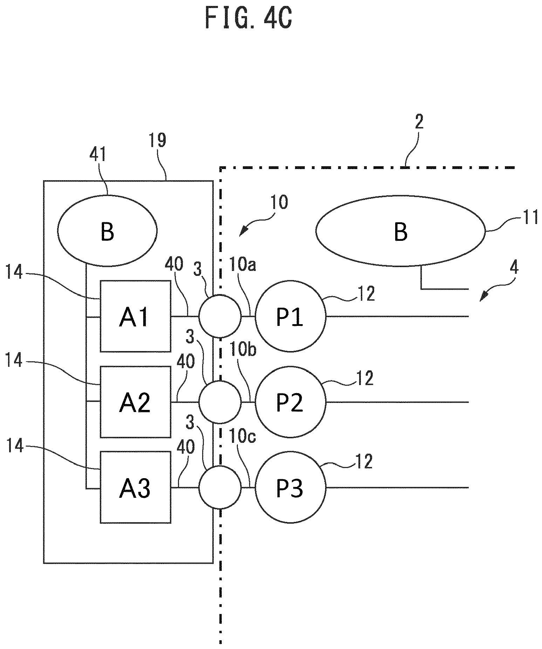

FIG. 4B and FIG. 4C illustrate one example of the fluid reservoir unit 14 provided in the fluid container 19. As an alternative embodiment, a fluid reservoir unit 14 and a dispense and suction unit 40 may be provided in the fluid container 19, separate from the cell production plate 2, and the fluid container 19 may be connected to at least one of a plurality of closed type connectors 3 to enable fluid to be dispensed into or suctioned from the fluid circuit 4 via the closed type connectors 3. The fluid container 19 may include a fluid reservoir unit 14 capable of storing at least one of a plurality of types of fluids, and a dispense and suction unit 40 capable of dispensing fluids into or suctioning fluids from the fluid circuit 4. One fluid container 19 with a plurality of dispense and suction units 40 may be connectable to a plurality of closed type connectors 3, and in this case, it is preferable to mount the plurality of closed type connectors 3 on one surface of the cell production plate 2. Furthermore, by providing the transfer unit 12 in the fluid circuit 4 or the fluid container 19 and enabling transfer of stored fluid into the fluid circuit 4, it becomes possible to transfer a predetermined amount of a specific stored fluid at an appropriate timing. This can simplify the process of injecting and discharging fluid by human resources or robots. The fluid reservoir unit 14 may be composed of a variable volume member, such as a syringe or a flexible bag, as illustrated in FIG. 4B, or a volume constant member, such as a rigid container, as illustrated in FIG. 4C. In the latter case, a second variable volume unit 41 in fluid communication with these fluid reservoir units 14 is provided in the fluid container 19, and the fluid originally contained in the second variable volume unit 41 is withdrawn into the fluid reservoir unit 14 or the fluid originally contained in the fluid reservoir unit 14 is extruded into the second variable volume unit 41, thereby enabling the movement of fluid within the fluid container 19 while maintaining the sealability of the fluid container 19.

The fluid mixing unit 15 includes a mixing flow path that mixes a plurality of mutually immiscible fluids. The mixing flow path preferably includes a fluid merging path and a mixed flow generation path. The fluid merging path is a path that merges mutually immiscible fluids into a single path, and the mixed flow generation path is a path that generates a mixed flow in the merged fluids. For example, the mixed flow generation path may be a spiral flow path that passes from the front side to the back side of the flat plate. In order to return the fluid from the back side of the flat plate to the front side, two spiral flow paths penetrating the flat plate are provided, and a communication path in fluid communication between these spiral flow paths is provided on the back side of the flat plate.

The cell separation unit 16 includes separation tanks D1-D2 for separating cells or cell masses. For example, the separation tanks D1-D2 are reservoir tanks formed by relatively increasing the width or depth of the flow paths, and the first separation tank D1 separates fluid containing only source cells from fluid containing source cells, and the second separation tank D2 allows only relatively large cell masses to settle and separate from the rest of the cell masses. As a method of separating the source cells, reagents for source cell separation, panning, magnetic cell separation (MACS), flow cytometry, or the like can be used.

The cell mass disruption unit 17 includes a disruption flow path that further disrupts separated cell masses (mass of one or more cells). The disruption flow path has a relatively small flow path area compared to the upstream flow path, and is preferably meandering. By meandering the flow path, a latent flow is generated, which applies shearing stress to the cell mass and breaks up a large growing cell mass into smaller cell masses. Latent flow is, for example, any of the following: flow that produces whirlpools, turbulence, reverse flow, flow that produces portions with different flow speeds, flow that produces shearing forces, and flow that produces portions where flows with different directions of travel collide.

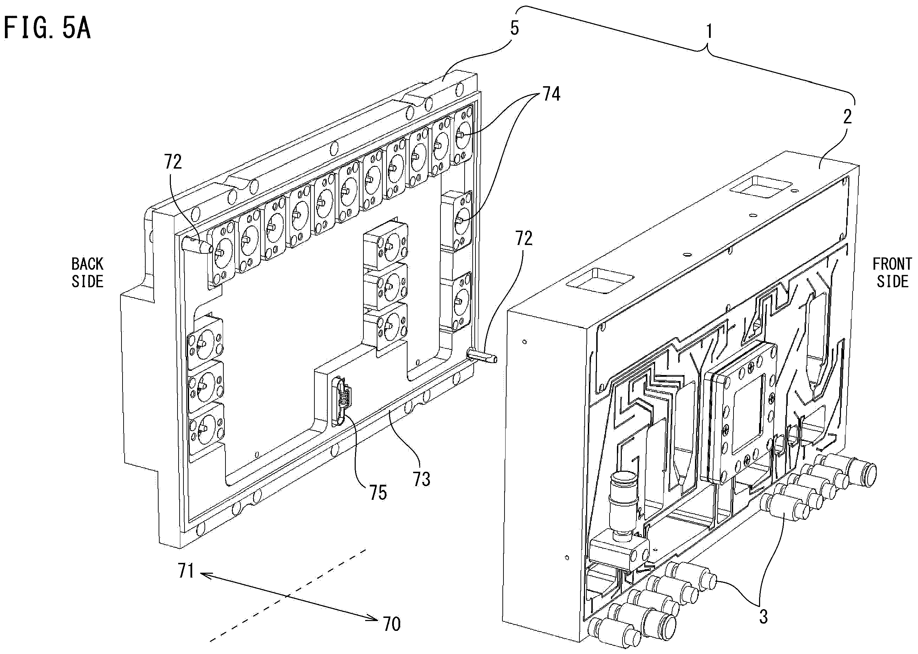

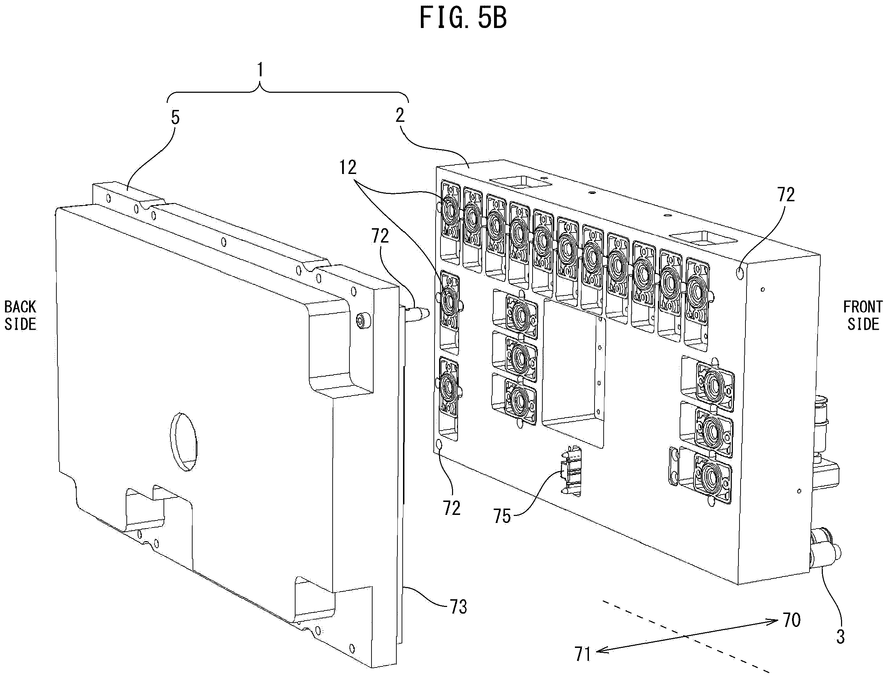

The cell production device 1 preferably further includes a base plate that is removably connected to the back side of the cell production plate 2. FIG. 5A and FIG. 5B illustrate one example of the base plate. The base plate 5 provides fluid control, temperature control, and the like for the cell production plate 2. The cell production plate 2 is arranged to face a dangerous area side 70 where robots and the like act, whereas the base plate 5 is arranged to face a safe area side 71 opposite to the dangerous area side 70. From the viewpoint of preventing biological contamination, the cell production plate 2 may be disposable and the base plate 5 may be reusable. The cell production device 1 is configured to be maintainable from the safe area side 71. When the cell production device 1 has such a two-sided structure, robots and the like can engage with a plurality of cell production devices 1 on a one-to-many basis.

The cell production device 1 may further include a positioning member 72 and a plate sealing member 73 on connecting surfaces of the cell production plate 2 and the base plate 5. The positioning member 72 may be a convex portion and a concave portion that fit each other, and positions the connection position of the cell production plate 2 and the base plate 5. The plate sealing member 73 may be a gasket, packing, or the like attached to the outer circumference of the connecting surface, and by connecting the cell production plate 2 and the base plate 5, the inside of the plate sealing member 73 is shut off from the external space and gas permeation from the back side of the cell production plate 2 is inhibited. The base plate 5 includes a drive unit 74 that drives the transfer unit 12. The drive unit 74 includes a motor that drives a peristaltic pump, for example. Furthermore, the connecting surface of the cell production plate 2 and the base plate 5 preferably includes electrical contacts 75 that supply power to electrical elements to be placed on the cell production plate 2, such as warming elements, cooling elements, flow sensors, and the like.

According to the above-described embodiment, since the cell production plate 2 is capable of storing a plurality of types of fluids or the fluid container 19 is capable of dispensing stored fluid to the cell production plate 2, it is possible to utilize a specific fluid in a predetermined amount at an appropriate timing even in the cell production device 1 that integrates a plurality of functional sites, thereby simplifying the process of injecting and discharging fluids by human resources or robots.

Although various embodiments have been described herein, it is to be recognized that the present invention is not limited to the various embodiments described above, and that various changes can be made within the scope of the following claims.

### REFERENCE SIGNS LIST

1 Cell production device
2 Cell production plate
3 Closed type connector
4 Fluid circuit
5 Base plate
10 Injection and discharge unit
10a-10f First-sixth injection and discharge channel
11 First variable volume unit
12 Transfer unit
13 Cell induction and culture unit
13a Cell induction and culture tank
13b Culture medium circulation path
13c pH measurement unit
14 Fluid reservoir unit
15 Fluid mixing unit
16 Cell separation unit
17 Cell mass disruption unit
19 Fluid container
20 Flat plate
20a Groove
20b Reservoir tank
20c Bank
21 Lid
21a Front lid
21b Back lid
30 Culture tank
31 Culture medium tank
32 Specific component-permeable member
40 Dispense and suction unit
41 Second variable volume unit
70 Dangerous area side
71 Safe area side
72 Positioning member
73 Plate sealing member
74 Drive unit
75 Electrical contact
A1-A4 First-fourth reservoir tank
D1-D2 First-second separation tank
P1-P8 First-eighth pump
S External space

## Claims

1. A cell production device (1) comprising
a cell production plate (2) that includes a fluid circuit (4) in which a plurality of functional sites are integrated, wherein
the fluid circuit (4) comprises, as the plurality of functional sites,
a plurality of fluid reservoir units (14) capable of storing a plurality of types of fluids and
a cell induction and culture unit (13) that is configured to perform induction and culture of cells based on the plurality of types of stored fluids, and
the fluid reservoir unit (14) stores at least one of a fluid containing source cells, a reagent for cell separation, a reagent for introducing an inducing factor, a culture medium for initialization or induction, and a fluid containing target cells

2. The cell production device (1) according to claim 1 , further comprising a first variable volume unit (11) configured to store the fluid to be extruded or withdrawn by injecting or discharging the plurality of types of fluids.

3. The cell production device (1) according to claim 2, wherein the first variable volume unit (11) comprises a physical or chemical variable volume material.

4. The cell production device (1) according to claim 3, wherein the physical variable volume material is a flexible bag or a syringe.

5. The cell production device (1) according to any one of claims 1 to 4, wherein the fluid circuit (4) further comprises a plurality of transfer units (12) capable of transferring the plurality of stored fluids.

6. The cell production device (1) according to any one of claims 1 to 5, wherein the plurality of closed type connectors (3) are mounted on one face of the cell production plate (2) and are connectable to a single fluid container (19) with a plurality of dispense and suction units (40).

7. A cell production system comprising:
a cell production device (1) comprising a cell production plate (2) that includes a fluid circuit (4) integrating a plurality of functional sites, and a plurality of closed type connectors (3) that connects the fluid circuit (4) to an external space (S) in a closed manner and, as the plurality of functional sites, a cell induction and culture unit (13); and
a fluid container (19) capable of being connected to at least one of the plurality of closed type connectors (3), wherein
the fluid circuit (4) comprises
a plurality of injection and discharge units (10) capable of injecting or discharging a plurality of types of fluids into or out of the fluid circuit (4) via the plurality of closed type connectors (3), and
the fluid container (19) comprises
a fluid reservoir unit (14) capable of storing at least one of the plurality of types of fluids, and
a dispense and suction unit (40) that dispenses and suctions fluids into or out of the fluid circuit (4) via at least one of the plurality of closed type connectors (3).

8. The cell production system according to claim 7, wherein the fluid circuit (4) further comprises a first variable volume unit (11) that is configured to store the fluid that is extruded or withdrawn by the injected or discharged fluid.

9. The cell production system according to claim 8, wherein the first variable volume unit (11) comprises a physical or chemical variable volume material.

10. The cell production system according to any one of claims 7 to 9, wherein the fluid container (19) further comprises a second variable volume unit (41) that is configured to store the fluid that is withdrawn and extruded by the dispensed and suctioned fluid.

11. The cell production system according to claim 10, wherein the second variable volume unit (41) comprises a physical or chemical variable volume material.

12. The cell production device (1) according to claim 9 or 11, wherein the physical variable volume material is a flexible bag or a syringe.

13. The cell production system according to any one of claims 7 to 12, wherein the fluid circuit (4) or the fluid container (19) further comprises a transfer unit (12) capable of transferring the stored fluid into the fluid circuit (4).
